# EUROPEAN PATENT SPECIFICATION

(11) **EP 3 976 018 B1**
(45) Date of publication and mention of the grant of the patent: **28.08.2024**
(21) Application number: 20814805.6
(22) Date of filing: 06.05.2020
(51) Int. Cl.: A61K 31/167, A61K 31/245, A61K 31/14, A61P 23/02, A61P 31/02, A61P 31/04, A61P 31/10, A61P 31/12

(54) **COMPOSITION FOR USE IN THE TREATMENT OF FOOT AND MOUTH DISEASE (FMD), SCABBY MOUTH (ORF), PICORNAVIRUS, PARAPOXVIRUS, AND A PATHOGENIC DISEASE CAUSING POX LESIONS**
ZUSAMMENSETZUNG ZUR VERWENDUNG BEI DER BEHANDLUNG DER MAUL- UND KLAUENSEUCHE (MKS), DER ORF, DES PICORNAVIRUS, DES PARAPOXVIRUS UND EINER PATHOGENEN KRANKHEIT, DIE POCKENLÄSIONEN VERURSACHT
COMPOSITION POUR UTILISATION DANS LE TRAITEMENT DE LA FIÈVRE APHTEUSE, DE L'ORF, DU PICORNAVIRUS, DU PARAPOXVIRUS ET D'UNE MALADIE PATHOGÈNE PROVOQUANT DES LÉSIONS DE LA VÉROLE

(30) Priority: 28.05.2019 AU 2019901824; 28.05.2019 AU 2019901837
(43) Date of publication of application: 06.04.2022
(73) Proprietor: Animal Ethics Pty Ltd, Yarra Glen, Victoria 3775 (AU)
(72) Inventor: OLSSON, Charles Robert, Crestmead, Queensland 4132 (AU); SHEIL, Meredith, Hunters Hill, New South Wales 2110 (AU); GIFFARD, Allan, Yarra Glen, Victoria 3775 (AU); WINDSOR, Peter, Scarborough, New South Wales 2515 (AU)
(74) Representative: Ernest Gutmann - Yves Plasseraud S.A.S.
(86) International application number: PCT/AU2020/050447
(87) International publication number: WO 2020/237284

(56) References cited:
- WO-A1-2006/096913
- WO-A1-2006/096913
- WO-A1-2010/006537
- WO-A1-2014/159731
- WO-A1-2014/159731
- WO-A1-93/00114
- US-A- 4 147 770
- US-A- 4 147 770
- US-A1- 2009 062 315
- US-A1- 2009 062 315
- MISK N A ET AL: "ASSESSMENT AND TOPICAL TREATMENT OF LESIONS OF FOOT AND MOUTH DISEASE IN CATTLE", ASSIUT VET. MED. J, vol. 61, 1 April 2015 (2015-04-01), pages 75 - 81, XP093024128
- ANAESTHESIA UK: "pH and local anaesthetics", 16 November 2009 (2009-11-16), XP093023789, Retrieved from the Internet <URL:https://www.frca.co.uk/article.aspx?articleid=220>
- JOHNSON SVENA M ET AL: "Local anesthetics as antimicrobial agents: a review", SURGICAL INFECTIONS, MARY ANN LIEBERT, LARCHMONT, NY, US, vol. 9, no. 2, 1 April 2008 (2008-04-01), pages 205 - 213, XP009148578, ISSN: 1096-2964
- PINA-VAZ CIDALIA ET AL: "Antifungal Activity of Local Anesthetics Against Candida Species", INFECTIOUS DISEASES IN OBSTETRICS AND GYNECOLOGY, vol. 8, no. 3-4, 1 January 2000 (2000-01-01), US, pages 124 - 137, XP055801764, ISSN: 1064-7449, DOI: 10.1155/S1064744900000168
- "Litman's Basics of Pediatric Anesthesia", 1 January 2022, ELSEVIER, ISBN: 978-0-323-82902-1, article KRAEMER F. WICKHAM: "Local Anesthetics and Adjuvant Analgesics", pages: 256 - 259, XP093099575, DOI: 10.1016/B978-0-323-82902-1.00034-2
- ANONYMOUS: "Local Anesthetics", BASICMEDICAL KEY, 14 August 2016 (2016-08-14), Basicmedical key - Fastest Basicmedical Insight Engine, XP093099732, Retrieved from the Internet <URL:https://basicmedicalkey.com/local-anesthetics-5/> [retrieved on 20231109]

## Description

### Related Applications

This application claims priority of Australian Provisional Patent Application No. 2019901824, filed 28 May 2019, and Australian Provisional Patent Application No. 2019901837, filed 28 May 2019.

### Technical Field

This invention relates to a topical composition for use in the treatment of a pathogenic disease caused by an acid-labile pathogen selected from foot and mouth disease (FMD), scabby mouth (Orf), Picornavirus, Parapoxvirus, and a pathogenic disease causing pox lesions. The invention also relates to a topical composition for use in the treatment of a diseased area of a subject caused by or aggravated by an acid-labile pathogen selected from the group consisting of foot and mouth disease (FMD), scabby mouth (Orf), Picornavirus, Parapoxvirus, and a pathogenic disease causing pox lesions.

### Background of the Invention

Foot and mouth disease (FMD), also known as hoof and mouth disease (HMD), is a highly contagious degenerative viral disease of cloven-hoofed animals such as cattle, buffalo, sheep, swine, goats, antelope, deer and bison. It is caused by a Picornavirus, and its symptoms include fever, loss of appetite and weight, blistering or boils on mucous membranes, particularly those of the mouth, feet and udder, animals walking in short tripping steps, lameness (hoof disease), and death.

Infection occurs when the virus is taken into a cell of the animal. The cell then manufactures copies of the virus and eventually bursts, releasing thousands of virus particles. Discharge from blisters on the skin, cavity of the mouth and fissures of the hoof is heavily infected with the virus. The disease is readily transmitted by contact or as an aerosol.

There is no effective treatment for FMD. Recovery rates are slow, animals may fail to recover, and infected animals may be slaughtered. An animal surviving the disease will acquire immunity for several months but only against the specific virus strain with which it was infected. Likewise, vaccines are only effective against specific virus strains.

The disease is most prevalent in Asia, Africa, and South America. Infected animals are usually quarantined and slaughtered together with healthy ones so as to limit contagion. The entire farm is then usually subjected to thorough disinfection.

Scabby mouth, also known as contagious pustular dermatitis, contagious ecthyma and orf, is a viral disease of goats and sheep. It is caused by a Parapoxvirus, and its symptoms include scabs and pustules, usually around the mouth and face of affected animals. Most commonly, raised scabs with a red ulcerated area underneath the scab occur around the lips, muzzle and nostril. Less commonly, raised scabs and ulcerated areas occur around the eyes, feet, lower leg, anus, vulva, udder, scrotum and pizzle. In severe cases, infected animal may stop eating, animals may become lame and animals with udder lesions may develop mastitis.

Abrasions in the skin enable virus to infect. Infection may also occur upon prolonged wetting of the feet. The disease can be transmitted by contact from sheep to sheep. More commonly, scabs shed from infected sheep spread the disease.

There is no specific treatment for scabby mouth, but infected animals usually recover spontaneously, with scabs healing in about 3-4 weeks. Animals can develop immunity provided that there is regular exposure to the virus. Animals can be vaccinated against the virus, usually for a 12-month period. The disease is prevalent in all sheep-raising countries.

Hoof rot/footrot is a catch-all term for a fungal or bacterial infection in the hoof of an animal, such as sheep, goats, cattle, horses and deer.

Footrot is caused by a combination of the bacteria *Fusobacterium necrophorum* and *Dichelobacter nodosus* (more common in sheep), and *Bacteroides melaninogenicus* (more common in cattle). Its symptoms include a foul-smelling necrotic lesion of the interdigital skin, swelling, reduced weight gain, decreased milk and wool production, and lameness. It is extremely painful and contagious.

Irritation of the interdigital area provides entry of the bacteria for infection. The disease is transmitted primarily from foot to foot via moist pastures, laneways and muddy yards.

Footrot is treated by foot bathing, hoof trimming, use of antibiotics and vaccination.

Animals that have been infected with or exposed to footrot do not develop any significant natural immunity or resistance. Vaccines have been developed, but their efficacy is unreliable and the immunity they provide is of short duration. A publication by Misk et al. (N. A. Misk, T. N. Misk and H. Z. Rateb, "Assessment and topical treatment of lesions of foot and mouth disease in cattle." Assiut Vet. Med. J., Vol.61, No. 145, April 2015) discloses the topical treatment of lesions of foot and mouth disease in cattle and mentions spraying a composition comprising a mixture of gentian violet, lidocaine, metronidazole and oxytetracycline on the lesions of subjects having the disease.

The product Tri-Solfen^{™} (Animal Ethics Pty Ltd) is a local anaesthetic and antiseptic gel spray for use on animals to provide pain relief following animal husbandry procedures such as mulesing, tail docking and castration. The product contains two topical local anaesthetics, being fast-acting lignocaine for immediate pain relief and long-acting bupivacaine for prolonged pain relief. The product's gel base adheres well to open wounds and acts as a barrier to environmental stimuli to improve wound healing. The Tri-Solfen^{™} product contains adrenalin, to both localise the anaesthetic effect and prevent or minimize systemic absorption, and is of acidic pH. See US Patent Nos. 8,960,128, 8,822,416 and 9,592,318 (Animal Ethics Pty Ltd).

### Detailed Description of the Invention

The present invention is defined by the claims. Any subject-matter falling outside the scope of the claims is provided for information only. Methods of treatment of the human or animal body by therapy, referred to in this description, are not part of the present invention as such, but are described here in relation to compounds and pharmaceutical compositions for use in said methods for treatment of the human or animal body by therapy, according to the present invention.

The present inventors have made a surprising discovery that the Tri-Solfen^{™} product is particularly effective in treating and controlling foot and mouth disease (FMD) in cattle when applied to a diseased area of the animal. Although it was anticipated that the gel-based product might alleviate pain caused by the virus when applied to FMD blistering and boils, what was not anticipated was that animals treated with the Tri-Solfen^{™} product would show markedly hastened healing of their lesions as well as rapidly regain their appetite, gain weight, overcome lameness and survive the disease.

The inventors postulate that the Tri-Solfen^{™} product provided immediate pain relief and, in addition, displayed unexpected viricidal properties, both of which contributed to rapid animal recovery from the disease.

The inventors postulate that the Tri-Solfen^{™} product exposed the virus to viricidal properties of the product, including acidic pH, with immediate effect, as well as for a prolonged period of time, enabled by the concomitant numbing of tissues by the local anaesthetic agents which prevented acid burning or stinging effect, and further effectively coated blistering and boils to lower the potential for virus discharge and transmission.

The inventors found that an anaesthetic agent could function as an acidic biocidal agent, yet any pain or discomfort caused by the acidic nature of the agent/composition was quickly countered by the fast-acting anaesthetic agent itself.

In view of the surprising efficacy of the Tri-Solfen^{™} product, the inventors postulate that similar compositions having both rapidly acting pain-relieving and viricidal properties can be used to treat and control FMD.

Based on the findings in cattle with FMD, the inventors have identified the potential to treat other infectious or inflammatory disease caused by a pathogen that results in epithelial or mucosal inflammation, ulceration, sloughing, peeling, excoriation or lesions such as vesicles or blisters that may rupture and form ulcers.

The inventors postulate that the Tri-Solfen^{™} product and like products can be used to treat and control other foot or mouth-type pathogenic diseases, including scabby mouth and hoof rot/footrot/foot abscess.

The inventors also postulate that the Tri-Solfen^{™} product and like products could be used to treat and control other bacterial, viral or fungal pathogens, particularly acid-labile pathogens that result in epithelial lesions or ulcerations of the skin or mucous membranes such as Herpesvirus lesions including shingles, cold-sores and genital herpes, and diseases caused by Rhinovirus and Horse Equine Rhinitis A virus (ERAV), Candida such as oral candidiasis, and Tinea.

The inventors also postulate that the Tri-Solfen^{™} product and like products could be used to treat and control infestational pathogens such as Lucilia cuprina blowfly larvae.

Flystrike lesions are very painful, being caused by the blowfly larvae. Larvae are killed by 0.5% hydrochloric acid. Applying acidic treatment however is likely to be painful. Therefore, a product like Tri-Solfen^{™} may inactivate larvae while also preventing or treating pain. Flystrike lesions may occur as examples in the breech, perineum, tail, poll pizzle or body of sheep.

According to a first aspect of the present invention, there is provided a topical composition for use in the treatment of a pathogenic disease caused by an acid-labile pathogen selected from foot and mouth disease (FMD), scabby mouth (Orf), Picornavirus, Parapoxvirus, and a pathogenic disease causing pox lesions, characterised in that:
the composition has an acidic pH;
the composition is applied to a subject at therapeutically effective amounts; and
the composition comprises:
   (a) Composition 1 comprising:
      about 100 mg/ml non-crystallising liquid sorbitol (70%);
      about 50.0 mg/ml lignocaine HCl;
      about 5.0 mg/ml bupivacaine HCl;
      about 1.5 mg/ml sodium metabisulfite;
      about 5.0 mg/ml cetrimide;
      about 45.0 µg/ml adrenaline tartrate;
      about 5.0 mg/ml hydroxy cellulose; and optionally colourant;
   (b) Composition 2 whereby lignocaine of Composition 1 is replaced by tetracaine at about 10 mg/ml - 100 mg/ml;
   (c) Composition 3 comprising:
      about 100 mg/ml non-crystallising liquid sorbitol (70%);
      about 40.0 mg/ml lignocaine HCl;
      about 1.5 mg/ml sodium metabisulfite;
      about 5.0 mg/ml cetrimide;
      about 36.0 µg/ml adrenaline tartrate;
      about 5.0 mg/ml hydroxy cellulose; and optionally
      colourant;
   (d) Composition 4 whereby lignocaine of Composition 3 is replaced by tetracaine at about 10 mg/ml - 100 mg/ml;
   (e) Composition 5 comprising:
      about 100.0 mg/ml purified water sorbitol liquid 70% non-crystallising;
      about 50.0 mg/ml (5%) tetracaine HCl;
      about 1.5 mg/ml sodium metabisulfite;
      about 5.0 mg/ml cetrimide;
      about 45.0 µg/ml adrenaline tartrate;
      about 5.0 mg/ml hydroxy cellulose;
      to 1 ml purified water; and optionally
      colourant (quantity to suit);
   (f) Composition 6 comprising: lignocaine, bupivacaine, adrenaline, cetrimide, 2-hydroxyethyl cellulose, sodium metabisulfite, liquid sorbitol (70%), buffer, and, optionally colourant;
   (g) Composition 7 whereby lignocaine of Composition 6 is replaced by tetracaine at about 10 mg/ml - 100 mg/ml;
   (h) Composition 8 comprising: amethocaine / tetracaine, adrenaline, cetrimide, 2-hydroxyethyl cellulose, sodium metabisulfite, liquid sorbitol (70%), buffer, and, optionally, colourant;
   (i) Composition 9 comprising a liquid gel matrix that contains the following:
      lidocaine; adrenalin; and cetrimide, and the composition is optionally coloured, wherein said composition has a pH lower than about 4.0;
   (j) Composition 10 comprising a liquid gel matrix that contains the following:
      tetracaine; adrenalin; and cetrimide, and the composition is optionally coloured, wherein said composition has a pH lower than about 4.0; or
   (k) Composition 11 comprising a liquid gel matrix that contains the following:
      lidocaine; bupivacaine; adrenalin; and cetrimide, and the composition is optionally coloured, wherein said composition has a pH lower than about 4.0.

According to a second aspect of the present invention, there is a topical composition for use in the treatment of a diseased area of a subject caused by or aggravated by an acid-labile pathogen selected from the group consisting of foot and mouth disease (FMD), scabby mouth (Orf), Picornavirus, Parapoxvirus, and a pathogenic disease causing pox lesions, characterised in that: the composition has an acidic pH;
the composition is applied to the diseased area; and
the composition comprises:
   (a) Composition 1 comprising:
      about 100 mg/ml non-crystallising liquid sorbitol (70%);
      about 50.0 mg/ml lignocaine HCl;
      about 5.0 mg/ml bupivacaine HCl;
      about 1.5 mg/ml sodium metabisulfite;
      about 5.0 mg/ml cetrimide;
      about 45.0 µg/ml adrenaline tartrate;
      about 5.0 mg/ml hydroxy cellulose; and optionally
      colourant;
   (b) Composition 2 whereby lignocaine of Composition 1 is replaced by tetracaine at about 10 mg/ml - 100 mg/ml;
   (c) Composition 3 comprising:
      about 100 mg/ml non-crystallising liquid sorbitol (70%);
      about 40.0 mg/ml lignocaine HCl;
      about 1.5 mg/ml sodium metabisulfite;
      about 5.0 mg/ml cetrimide;
      about 36.0 µg/ml adrenaline tartrate;
      about 5.0 mg/ml hydroxy cellulose; and optionally
      colourant;
   (d) Composition 4 whereby lignocaine of Composition 3 is replaced by tetracaine at about 10 mg/ml - 100 mg/ml;
   (e) Composition 5 comprising:
      about 100.0 mg/ml purified water sorbitol liquid 70% non-crystallising;
      about 50.0 mg/ml (5%) tetracaine HCl;
      about 1.5 mg/ml sodium metabisulfite;
      about 5.0 mg/ml cetrimide;
      about 45.0 µg/ml adrenaline tartrate;
      about 5.0 mg/ml hydroxy cellulose;
      to 1 ml purified water; and optionally colourant (quantity to suit);
   (f) Composition 6 comprising: lignocaine, bupivacaine, adrenaline, cetrimide, 2-hydroxyethyl cellulose, sodium metabisulfite, liquid sorbitol (70%), buffer, and, optionally colourant;
   (g) Composition 7 whereby lignocaine of Composition 6 is replaced by tetracaine at about 10 mg/ml - 100 mg/ml;
   (h) Composition 8 comprising: amethocaine / tetracaine, adrenaline, cetrimide, 2-hydroxyethyl cellulose, sodium metabisulfite, liquid sorbitol (70%), buffer, and, optionally, colourant;
   (i) Composition 9 comprising a liquid gel matrix that contains the following:
      lidocaine; adrenalin; and cetrimide, and the composition is optionally coloured, wherein said composition has a pH lower than about 4.0;
   (j) Composition 10 comprising a liquid gel matrix that contains the following:
      tetracaine; adrenalin; and cetrimide, and the composition is optionally coloured, wherein said composition has a pH lower than about 4.0; or
   (k) Composition 11 comprising a liquid gel matrix that contains the following:
      lidocaine; bupivacaine; adrenalin; and cetrimide, and the composition is optionally coloured, wherein said composition has a pH lower than about 4.0.

In the context of the present invention, the pathogenic disease is preferably scabby mouth (Orf) or foot and mouth disease (FMD).

In each of the aspects, the composition or medicament has both pain-relieving and biocidal properties. In each of the aspects, the composition or medicament has rapid pain-relieving properties.

In some embodiments, the diseased area is the skin or mucous membrane of the subject.

In some embodiments, the diseased area corresponds to epithelial or mucosal denuding, inflammation, excoriation, ulceration, sloughing, peeling or lesions such as pustules, pox lesions, eruptions, vesicles or blisters that may rupture and form ulcers.

Preferably the diseased area corresponds to significant epithelial or mucosal denuding, inflammation, excoriation, ulceration, sloughing, peeling or lesions such as pustules, pox lesions, eruptions, vesicles or blisters that may rupture and form ulcers, as opposed to minor epithelial or mucosal denuding, inflammation, excoriation, ulceration, sloughing, peeling or lesions such as pustules, pox lesions, eruptions, vesicles or blisters that may rupture and form ulcers. That is, for example, the composition is used on significant pathogenic ulcerative lesions, as opposed to minor pathogenic ulcers or skin excoriation.

In some embodiments the diseased area corresponds to a mouth and/or nasal area of the subject. In some embodiments the diseased area corresponds to the genital and/or anal area of a subject. In some embodiments the diseased area corresponds to a hand, arm, foot, hoof or leg of the subject. In some embodiments the diseased area corresponds to a face or thorax (including abdomen and back) of a subject. In some embodiments the diseased area corresponds to perineum, armpits, under folds of the breasts, in creases or folds of skin on the abdomen etc of a subject. In some embodiments the diseased area corresponds to feet, scalp, skin, or skin folds or creases of a subject. In some embodiments the diseased area corresponds to lesions in the breech, perineum, tail, poll pizzle or body of a subject.

The composition (or medicament) can be of any suitable form. The composition can be, for example, in the form of a liquid, ointment, gel, lotion, cream, crème, emulsion, paste, film or suspension.

Depending on the form of the composition, the composition can include one or more of the following types of ingredients: adhesive; aqueous or oily diluent; carrier; excipient; base; buffer; pH adjuster; bittering agent (i.e. foul-tasting agent); suspending agent; thickening agent; gelling agent; viscosity increasing agent; emulsifier; emollient; humectant; stabilising agent; dispersing agent/dispersant; solubiliser; skin conditioning agent; skin protectant; skin penetration enhancer; fragrance; preservative; sunscreen agent; surfactant; textural modifier; colourant; propellant; refrigerant; and, waterproofing agent.

Suitable oily or aqueous bases, carriers, diluents and excipients are inert and physiologically acceptable and include, for example: bacteriostatic saline (saline containing benzyl alcohol), cetomacrogol, cetyl alcohol, glycerine, lanolin, petrolatum based creams, gels, hydrogels, saline, short chain alcohols and glycols (e.g. ethyl alcohol and propylene glycol), and water.

Either water in oil or oil in water emulsions can be used. Examples of suitable surfactants and emulsifying agents include: non-ionic ethoxylated and non-ethoxylated surfactants, abietic acid, almond oil PEG, beeswax, butylglucoside caprate, C₁₈-C₃₆ acid glycol ester, C₉-C₁₅ alkyl phosphate, caprylic/capric triglyceride PEG-4 esters, cetomacrogol, ceteareth-7, cetereth-20, cetyl phosphate, cetyl stearyl alcohol, corn oil PEG esters, DEA-cetyl phosphate, dextrin laurate, dilaureth-7 citrate, dimyristyl phosphate, glycereth-17 cocoate, glyceryl erucate, glycerol, glyceryl laurate, G.M.S. acid stable, hydrogenated castor oil PEG esters, isosteareth-11 carboxylic acid, lecithin, lysolecithin, nonoxynol-9, octyldodeceth-20, palm glyceride, PEG diisostearate, PEG stearamine, poloxamines, polyglyceryls, potassium linoleate, PPGs, raffinose myristate, sodium caproyl lactylate, sodium caprylate, sodium cocoate, sodium isostearate, sodium tocopheryl phosphate, steareths, TEA-C₁₂-C₁₃ pareth-3 sulfate, tri-C₁₂-C₁₅ pareth-6 phosphate, and trideceths.

The composition can comprise one or more types of preservative. A suitable preservative, for example, can be: benzalkonium chloride, benzoic acid, benzothonium chloride, benzyl alcohol, 2-bromo-2-nitropropane-1,3-diol, bronopol, butylated hydroxyanisole, butylated hydroxytoluene, butyl paraben, chlorophene, chlorphenesin, diazolidinyl urea, DMDM hydantoin, ethyl paraben, formaldehyde-releasing preservative, hydroquinone, iodopropynyl butylcarbamate, imidazolidinyl urea, methyldibromo glutaronitrile, methylhydroquinone, methylisothiazolinone, methyl paraben, nitrosamines, o-cymen-5-ol, phenoxyethanol, propyl paraben, quaternium-15, sodium benzoate, sodium dehydroacetate, sodium hydroxymethylglycinate, sodium metabisulfite, and sodium sulfite. Preferably, the composition includes the reducing agent such as sodium metabisulfite. Any suitable amount of sodium metabisulfite can be used, eg. up to about 3.5mg/ml.

The composition can include a colourant so that its application can be verified visually. The colourant can be a pigment and/or dye. Suitable colourants include, for example, common food dyes or the ORCODERM^{®}, ORCOBRITE^{®} and ORCOFUR^{®} lines of pigments and dyes sold by the Organic Dyestuffs Corporation. Preferably, the colourant is non-toxic and will not permanently stain the skin or animal hide or surrounding hair, fur or wool.

A skin conditioning agent, as defined herein, improves dry or damaged skin. Such agents, for example, include: acetyl cysteine, N-acetyl dihydrosphingosine, acrylates/behenyl acrylate/dimethicone acrylate copolymer, adenosine, adenosine cyclic phosphate, adensosine phosphate, adenosine triphosphate, alanine, albumen, algae extract, allantoin and derivatives, aloe barbadensis extracts, aluminum PCA, amyloglucosidase, arbutin, arginine, azulene, bromelain, buttermilk powder, butylene glycol, caffeine, calcium gluconate, capsaicin, carbocysteine, carnosine, beta-carotene, casein, catalase, cephalins, ceramides, chamomilla recutita (matricaria) flower extract, cholecalciferol, cholesteryl esters, coco-betaine, coenzyme A, corn starch modified, crystallins, cycloethoxymethicone, cysteine DNA, cytochrome C, darutoside, dextran sulfate, dimethicone copolyols, dimethylsilanol hyaluronate, DNA, elastin, elastin amino acids, epidermal growth factor, ergocalciferol, ergosterol, ethylhexyl PCA, fibronectin, folic acid, gelatin, gliadin, beta-glucan, glucose, glycine, glycogen, glycolipids, glycoproteins, glycosaminoglycans, glycosphingolipids, horseradish peroxidase, hydrogenated proteins, hydrolyzed proteins, jojoba oil, keratin, keratin amino acids, kinetin, lactoferrin, lanosterol, lauryl PCA, lecithin, linoleic acid, linolenic acid, lipase, lysine, lysozyme, malt extract, maltodextrin, melanin, methionine, mineral salts, niacin, niacinamide, oat amino acids, oryzanol, palmitoyl hydrolyzed proteins, pancreatin, papain, PEG, pepsin, phospholipids, phytosterols, placental enzymes, placental lipids, pyridoxal 5-phosphate, quercetin, resorcinol acetate, riboflavin, RNA, saccharomyces lysate extract, silk amino acids, sorbitol, sphingolipids, stearamidopropyl betaine, stearyl palmitate, tocopherol, tocopheryl acetate, tocopheryl linoleate, ubiquinone, *vitis vinifera* (grape) seed oil, wheat amino acids, xanthan gum, and zinc gluconate.

The composition can include a skin penetration enhancer for enhancing the penetration of active ingredients. Any suitable type of enhancer can be used. Examples of suitable enhancers may include solvents, detergents or low carbon alcohols such as dimethylsulfoxide, oleyl alcohol, propylene glycol, methyl pyrrolidone and dodecylazyl cycloheptan 2-one.

The composition can comprise one or more of the following adhesives, thickening agents, gelling agents and/or viscosity increasing agents: acrylamides copolymer, agarose, amylopectin, calcium alginate, calcium carboxymethyl cellulose, carbomer, carboxymethyl chitin, castor oil derivatives, cellulose gum, cellulosic preparation, cetyl alcohol, cetostearyl alcohol, dextrin, gelatin, hydroxy cellulose, hydroxyethylcellulose, hydroxypropylcellulose, hydroxpropyl starch, inert sugar, magnesium alginate, methylcellulose, microcrystalline cellulose, pectin, PEG's, polyacrylic acid, polymethacrylic acid, polyvinyl alcohol, quaternium ammonium compound of bentonite or zinc stearate, sorbitol, PPG's, sodium acrylates copolymer, sodium carrageenan, xanthum gum, and yeast beta-glucan.

The composition can comprise an insecticide or insect repellent to stop insect infestation. Any suitable type of insecticide or insect repellent can be used. Examples of suitable insecticides include: trichlorfon, triflumeron, fenthion, bendiocarb, cyromazine, dislubenzuron, dicyclanil, fluazuron, amitraz, deltamethrin, cypermethrin, chlorfenbinphos, flumethrin, ivermectin, abermectin, avermectin, doramectin, moxidectin, zeti-cypermethrin, diazinon, spinosad, imidacloprid, nitenpyran, pyriproxysen, sipronil, cythioate, lufenuron, selamectin, milbemycin oxime, chlorpyrifos, coumaphos, propetamphos, alpha-cypermethrin, high *cis* cypermethrin, ivermectin, diflubenzuron, cyclodiene, carbamate and benzoyl urea.

The composition can be applied to the subject (or diseased area of the subject) in any suitable form. The composition can be applied in a liquid form or other free-flowing form. The composition can be applied as a spray-on liquid or spray-on gel so as to minimise pain related to touching a subject, minimise the risk of infection from skin contamination and so that the diseased area need not be disturbed more than necessary. Alternatively, the composition can be applied as a gel by hand, or squeezed from a tube.

In some embodiments the composition is adhesive or sticky or tenacious.

Once applied, the composition can be, for example, in the form of an ointment, gel, foam, lotion, crème, cream, emulsion, paste, solution or suspension, or may set and /or form a physical barrier, 'skin', coating or film.

Once applied, the composition can be, for example, in the form of an adhesive/sticky/tenacious ointment, gel, foam, lotion, crème, cream, emulsion, paste, solution or suspension, or may set and /or form an adhesive physical barrier, 'skin', coating or film.

For example, if applied to a diseased area such as a leg or hoof, the composition may form an adhesive coat or coating, layer, barrier, skin or film. That is, the composition can be adhesive.

For example, if applied to a diseased wet area such as a mouth and/or nasal region, the composition may foam and spread over those wet surfaces, or otherwise coat and spread over those wet surfaces.

The composition can be in a sustained-release form, whereby one or more actives of the composition are slowly released to provide a therapeutic effect over an extended period of time (eg. biocidal activity, pain relief, antiviral activity).

The composition can be incorporated into a bandage, plaster, dressing, wipe or tissue.

The composition can be applied as a metered dose.

The composition can form or can be in the form of an adhesive gel after being applied to the subject.

The composition can form or can be in the form of a foam after being applied to the subject.

The composition can be both adhesive/sticky in a relatively dry diseased area and/or form a foam in a relatively wet diseased area.

The composition can comprise a hydrophilic or hydroalcoholic gelling agent. The composition can comprise about 1 to 20 g per litre of at least one type of gum or cellulosic preparation, including all about 0.1 g increments between 1 and 20, including 1.1, 1.2 etc. The composition can comprise a polyhydric alcohol in combination with a cellulosic preparation; for example, hydroxy cellulose (eg. hydroxyethyl cellulose, ethylhydroxy cellulose) in combination with non-crystallising liquid sorbitol. The composition can comprise about 5 mg/ml hydroxy cellulose (eg. hydroxyethyl cellulose) in combination with about 100 mg/ml non-crystallising liquid sorbitol (70%).

Preferably, the composition is in the form of a liquid prior to having been applied to a subject. Preferably, the composition forms, or is in the form of, a sticky, viscous, adhesive gel when applied to a subject. Preferably, the composition is in the form of a spray-on gel that can coat the diseased area of the subject and can maximise delivery of actives to the diseased area of the subject, preferably by way of staying moist and viscous (i.e. "sticky").

Preferably, the composition forms, or is in the form of, a foam when applied to a subject. Preferably, the composition is in the form of a foam that can coat the diseased area of the subject and can maximise delivery of actives to the diseased area of the subject. Preferably the composition, as a foam, can spread throughout the diseased area.

Preferably, the composition forms an effective long-lasting barrier over the diseased area. The term "long-lasting barrier" is to be understood as meaning a barrier/seal that is substantially capable of remaining intact over a diseased area for hours, days, a week or even weeks, or until the wound has naturally sealed or the pain has otherwise abated by way of the natural healing process - eg. about 1, 2, 3, 4, 5, 6, 7, 8, 9, 10, 11, 12, 13, 14, 15, 16, 17, 18, 19, 20, 21, 22, 23 or 24 hours, or about one, two, three, four, five, six or seven days, or one, two, three, four or more weeks.

The barrier preferably aids in the healing process, presumably by minimising or preventing water loss from the diseased area and by acting as a barrier against microbial contamination.

In some embodiments the composition is in the form of a liquid that thickens to an adhesive gel when reacting with physiological fluids of the diseased area of the subject.

The composition can provide prolonged pain relief, including pain relief for about 0.5, 1, 2, 3, 4, 5, 6, 7, 8, 9, 10, 11, 12, 13, 14, 15, 16, 17, 18, 19, 20, 21, 22, 23 or 24 hours, or about one, two, three, four, five, six or seven days, or one, two, three, four or more weeks, or until the wound has naturally sealed or the pain has otherwise abated by way of the natural healing process.

The composition can provide prolonged pain relief, including pain relief for about 0.5, 1, 2, 3, 4, 5, 6, 7, 8, 9, 10, 11, 12, 13, 14, 15, 16, 17, 18, 19, 20, 21, 22, 23 or 24 hours, or about one, two, three, four, five, six or seven days, or one, two, three, four or more weeks.

The composition can provide prolonged release of a biocidal agent or exposure to a biocidal property, including for about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23 or 24 hours, or about one, two, three, four, five, six or seven days, or one, two, three, four or more weeks. The composition comprises both at least one local anaesthetic agent having a rapid onset of action and at least one local anaesthetic agent having a long duration of action. It is to be understood that some local anaesthetic agents can provide both a rapid onset of action and long duration of action, such as amethocaine / tetracaine, so the local anaesthetic agent providing a rapid onset of action and local anaesthetic agent providing a long duration of action can be one and the same.

Anaesthetic agents that usually have a rapid onset of action (usually between about 5-10 minutes) include lignocaine, prilocaine, amethocaine / tetracaine and cocaine.

Anaesthetic agents that often have a much slower onset of action but a much greater duration of action (usually between about 4-12 hours of anaesthesia) include bupivacaine, ropivacaine, levo-bupivacaine, and amethocaine / tetracaine. Bupivacaine may typically provide up to about 6-12 hours of anaesthesia, depending on the method of administration.

Any suitable amount of anaesthetic agent can be used in the composition but preferably about 0.01-10 weight/volume % of anaesthetic agent is used (as well as all 0.01 increments between 0.01 and 10, eg. 0.01, 0.02 etc).

Any suitable amount of rapid onset anaesthetic agent can be used in the composition but preferably about 0.01-10 weight/volume % of anaesthetic agent is used (as well as all 0.01 increments between 0.01 and 10). Preferably, about 2-8 weight/volume % anaesthetic agent is used in those situations where a rapid onset of action is required (as well as all 0.01 increments between 2 and 8). More preferably, about 5 % weight/volume anaesthetic agent is used.

In some embodiments, about 1-10 weight/volume % lignocaine is used (as well as all 0.01 increments between 1 and 10, eg. 0.01, 0.02 etc). In some embodiments, about 2-8 weight/volume % lignocaine is used as the anaesthetic agent in those situations where a rapid onset of action is required (as well as all 0.01 increments between 2 and 8). In some embodiments, about 5 % lignocaine is used.

Any suitable amount of long duration of action anaesthetic agent can be used in the composition but preferably about 0.01-10 weight/volume % of anaesthetic agent is used (as well as all 0.01 increments between 0.01 and 10). Preferably, about 0.1-5 weight/volume % anaesthetic agent is used in those situations where a long duration of action is required (as well as all 0.01 increments between 0.1 and 5). More preferably, about 0.5 % weight/volume anaesthetic agent is used.

In some embodiments, the composition can comprise any suitable amount of bupivacaine if lignocaine or other rapid onset anaesthetic agent has an inadequate duration of action. Preferably, the composition comprises about 0.1-5 weight/volume % bupivacaine (as well as all 0.01 increments between 0.1 and 5), and more preferably about 0.5% bupivacaine.

In some embodiments, the composition can comprise any suitable amount of tetracaine. Preferably, the composition comprises about 1-10 weight/volume % tetracaine (as well as all 0.01 increments between 1 and 10).

In some embodiments, at least one analgesic agent and/or at least one anti-inflammatory agent can be used. Examples of potentially suitable analgesic and/or anti-inflammatory agents include: 1-menthol, acetaminophen, alclofenac, aspirin, bendazac, betamethasone, bufexemacpiroxicam, camphor, choline salicylate, clidanac, clofezone, corticosterone, cortisone, dexamethasone, diclofenac, diflunisal, fenbufen, fenoprofen, fentiazac, fludrocortisone, fluocinolone, fluphenamic acid, flurandrenolide, flurbiprofen, glycol salicylate, halcinonide, hydrocortisone, ibuprofen, indomethacin, indoprofen, ketoprofen, meclofenamate sodium, mefenamic acid, medrysone, mepirizole, methylprednisolone, methyl salicylate, naproxene, oxyphenbutazone, paramethasone, pentazocine, phenylbutazone, piroxicam, prednisone, prednisolone, protizidic acid, pranoprofen, salicylic acid, sulindac, suprofen, tiaprofenic acid, tolmetin and triamcinolone.

Potentially suitable analgesic agents include one or more of the following: acetaminophen, aspirin, salicylic acid, methyl salicylate, choline salicylate, glycol salicylate, 1-menthol, camphor, mefenamic acid, fluphenamic acid, indomethacin, diclofenac, alclofenac, ibuprofen, ketoprofen, pranoprofen, fenoprofen, sulindac, fenbufen, clidanac, flurbiprofen, indoprofen, protizidic acid, fentiazac, tolmetin, tiaprofenic acid, bendazac, bufexemacpiroxicam, phenylbutazone, oxyphenbutazone, clofezone, pentazocine, mepirizole, hydrocortisone, cortisone, dexamethasone, fluocinolone, triamcinolone, medrysone, prednisolone, flurandrenolide, prednisone, halcinonide, methylprednisolone, fludrocortisone, corticosterone, paramethasone, betamethasone, naproxen, suprofen, piroxicam, diflunisal, meclofenamate sodium, carprofen, flunixin, tolfenamic acid and meloxicam.

Potentially suitable non-steroidal anti-inflammatory drug (NSAIDs) include one or more of the following: salicylate (e.g. aspirin (acetylsalicylic acid), diflunisal (dolobid), salicylic acid and other salicylates, salsalate (disalcid)), propionic acid derivative (e.g. ibuprofen, dexibuprofen, naproxen, fenoprofen, ketoprofen, dexketoprofen, flurbiprofen, oxaprozin, loxoprofen), acetic acid derivative (e.g. indomethacin, tolmetin, sulindac, etodolac, ketorolac, diclofenac, aceclofenac, nabumetone), enolic acid (oxicam) derivative (e.g. piroxicam, meloxicam, tenoxicam, droxicam, lornoxicam. isoxicam, phenylbutazone), anthranilic acid derivative (fenamate) (e.g. mefenamic acid, meclofenamic acid, flufenamic acid, tolfenamic acid), selective COX-2 inhibitor (e.g. celecoxib, rofecoxib, valdecoxib, parecoxib, lumiracoxib, etoricoxib, firocoxib), sulfonanilide (e.g. nimesulide), or other (e.g. clonixin, licofelone, H-harpagide in Figwort or Devil's Claw).

In some embodiments approximately 0.1% (1mg/ml) meloxicam or other NSAID is used.

Any suitable amount of analgesic or anti-inflammatory agent can be used in the composition but preferably about 0.01-10 weight/volume % of agent is used (as well as all 0.01 increments between 0.01 and 10).

The composition can further include a vasoconstrictor to decrease the rate of vascular absorption of the pain relieving agent, so to improve the depth and duration of pain relieving agent, to reduce bleeding from a wound of the subject, as well as to reduce systemic toxicity. The vasoconstrictor can also lower the pH. Any suitable type of vasoconstrictor can be used, including any suitable salt or form thereof. Suitable vasoconstrictors include, for instance, adrenaline (epinephrine), noradrenalin (norepinephrine) and fenylpressin, including any suitable salt or form thereof. Preferably, the composition includes about 1:1000-1:10,000 vasoconstrictor (as well as all 10 factor increments between 1000 to 10,000), and more preferably 1:2,000 vasoconstrictor. Preferably the vasoconstrictor is adrenaline.

Regarding the biocidal properties of the composition or medicament, the composition or medicament can comprise at least one biocidal agent. However, depending on the type of pathogen/s, the composition or medicament can have biocidal properties attributable to any one or more of the following: low pH (below about pH 4.0, but more preferably below about pH 3.0); antiseptic agent; antimicrobial agent; viricidal agent (eg. at least one anaesthetic agent such as lidocaine, preferably in high doses); antifungal agent; antibiotic; and, vaccine antigen specific for the pathogen. (Local anaesthetic solutions typically have an acidic pH to maximise their water solubility and chemical stability.)

The composition can include one or more other active ingredients. An active ingredient, as defined herein, is a compound that provides benefit to the subject. The active ingredient can be, for instance, an anticoagulant, antiproliferative, cytokine, cytotoxin, growth factor, interferon, haemostatic agent, hormone, lipid, demineralized bone or bone morphogenetic protein, cartilage inducing factor, oligonucleotide, polymer, polysaccharide, polypeptide, protease inhibitor, vitamin, mineral, and, insecticide or insect repellent.

The composition can have a pH below about 4.0, which includes a pH of about 3.9, 3.8, 3.7, 3.6, 3.5, 3.4, 3.2, 3.1, 3.0 .2.9, 2.8, 2.7, 2.6, 2.5, 2.4, 2.3, 2.2, 2.1 and 2.0.

The composition preferably includes an antiseptic agent to, amongst other things, minimize wound contamination and infection, and act as an anti-viral agent. Any suitable type of antiseptic agent can be used. Suitable antiseptic agents include cetrimide, povidone-iodine, chlorhexidine, iodine, benzalkonium chloride, benzoic acid, nitrofurazone, benzoyl peroxide, hydrogen peroxide, hexachlorophene, phenol, resorcinol and cetylpyridinium chloride.

The subject can be a human. The subject can be an animal. The subject can be any member of the mammalian order Artiodactyla. The subject can be any relevant type of hoofed animal. The subject can be any relevant type of hoofed African animal. Hoofed animals include, for example: cattle, reindeer, giraffe, deer, sheep, moose, camel, hippopotamus, goat, pig, boar, elk, llama, bison, alpaca, okapi, yak, ruminants of the suborder Ruminantia, buffalo, gaur, antelope, gazelle, ox, gnu and suid. The animal is preferably a sheep, horse, cow, goat, pig, deer, antelope, bison or buffalo.

Preferably the composition is capable of coating and adhering to a diseased area of the subject.

Preferably the composition is capable of controlled and/ or prolonged release of a biocidal agent or exposure to a biocidal property.

Preferably the composition is capable of providing rapid pain relief. Preferably the composition is capable of providing prolonged pain relief. Preferably the composition is capable of providing both rapid pain relief and prolonged pain relief.

Preferably the composition is capable of providing rapid pain relief so as to relief pain caused by an acidic composition.

Preferably the composition is biocompatible and absorbable such that it does not require removal.

Preferably the composition comprises an adhesive gel base capable of coating and adhering to a diseased area and providing prolonged contact with the diseased area and controlled and/or prolonged release of a biocidal agent or exposure to a biocidal property.

In some embodiments, the biocidal property is that the composition comprises a pH less than about 3.0. In some embodiments, the biocidal agent is lignocaine. In some embodiments, the biocidal property is that the composition is acidic and can inactivate a pathogenic virus. In some embodiments, the biocidal agent is substantially uniformly dispersed throughout the composition. In some embodiments, the biocidal agent is an antiseptic, such as cetrimide. In some embodiments, the composition comprises a gelling agent or thickener, such as hydroxyethyl cellulose. In some embodiments, the composition comprises a gelling agent or thickener, such as non-crystallising liquid sorbitol (70%). In some embodiments, the composition comprises a colourant, such as a dye. In some embodiments, the composition comprises a pH less than about 4.0, preferably less than about 3.0. In some embodiments the composition is in the form of a liquid that sets as a sticky viscous gel when exposed to the diseased area.

In some embodiments, the composition comprises: a liquid gel matrix that contains the following: lidocaine; adrenalin; and cetrimide, and the composition is optionally coloured; or a liquid gel matrix that contains the following: tetracaine; adrenalin; and cetrimide, and the composition is optionally coloured; or a liquid gel matrix that contains the following: lidocaine; bupivacaine; adrenalin; and cetrimide, and the composition is optionally coloured, wherein said composition has a pH lower than about 4.0.

In a first preferred embodiment, the composition ("Composition 1") comprises:
about 100 mg/ml non-crystallising liquid sorbitol (70%);
about 50.0 mg/ml lignocaine HCl;
about 5.0 mg/ml bupivacaine HCl;
about 1.5 mg/ml sodium metabisulfite;
about 5.0 mg/ml cetrimide;
about 45.0 µg/ml adrenaline tartrate;
about 5.0 mg/ml hydroxy cellulose; and optionally
colourant such as a dye.

If desired, lignocaine can be swapped out and tetracaine swapped in at about 1-10%, but preferably about 5% (50mg/ml) ("Composition 2").

In a second preferred embodiment, the composition ("Composition 3") comprises:
about 100 mg/ml non-crystallising liquid sorbitol (70%);
about 40.0 mg/ml lignocaine HCl;
about 1.5 mg/ml sodium metabisulfite;
about 5.0 mg/ml cetrimide;
about 36.0 µg/ml adrenaline tartrate;
about 5.0 mg/ml hydroxy cellulose; and optionally
colourant such as a dye.

If desired, lignocaine can be swapped out and tetracaine swapped in at about 1-10%, but preferably about 5% (50mg/ml) ("Composition 4").

In a third preferred embodiment, the composition ("Composition 5") comprises:
about 100.0 mg/ml purified water sorbitol liquid 70% non-crystallising;
about 50.0 mg/ml (5%) tetracaine HCl;
about 1.5 mg/ml sodium metabisulfite;
about 5.0 mg/ml cetrimide;
about 45.0 µg/ml adrenaline tartrate;
about 5.0 mg/ml hydroxy cellulose;
to 1 ml purified water; and optionally
colourant such as a food dye (quantity to suit (q.s.)).

In a fourth preferred embodiment, the composition ("Composition 6") comprises: lignocaine, bupivacaine, adrenaline, cetrimide, 2-ethyl hydroxycellulose, sodium metabisulfite, liquid sorbitol (70%), buffer, and, optionally colourant such as a dye.

If desired, lignocaine can be swapped out and tetracaine swapped in at about 1-10%, but preferably about 5% (50mg/ml) ("Composition 7").

In a fifth preferred embodiment, the composition ("Composition 8") comprises: amethocaine / tetracaine, adrenaline, cetrimide, 2-ethyl hydroxycellulose, sodium metabisulfite, liquid sorbitol (70%), buffer, and, optionally, colourant such as a dye.

In a preferred embodiment, the composition ("Composition 9") is as described or substantially as described in US Patent Nos. 8,960,128, 8,822,416 and 9,592,318 (to Animal Ethics Pty Ltd).

Any suitable dosage and dosage regime can be used. This will depend on, amongst other things, the ingredients/actives/properties of the composition, the size/weight of the subject being treated, the size of the diseased area/lesion area being treated, and the frequency of reapplication of the composition that is required (if any).

Suitable dosages can be gleaned, for example, from the Best Mode section of this specification.

Preferably, the entire diseased area of the subject is coated with the composition, eg. hoof and mouth regions, in the case of foot and mouth disease. The amount applied will vary according to the number and size of the diseased areas/lesions.

In the case of Compositions 1 to 9 and similar compositions, in some embodiments this will usually amount to application of between about 5ml and 60ml of Composition per subject, including all 0.1 ml values between 5 and 60. However, more or less could be applied.

In the case of Compositions 1 to 9 and similar compositions, in some embodiments this will amount to application of between about 6ml and 20ml of Composition per subject, including all 0.1 ml values between 6 and 20. However, more or less could be applied.

For any one of the Compositions and similar compositions containing lidocaine but no adrenalin, about 2.6g lidocaine (without adrenalin) can be applied to cattle or other subjects weighing 600-800kg = approximately 3-4mg/kg.

The application dose of a said Composition (containing lidocaine plus adrenalin) may be up to about 50mg lidocaine/kg of subject body weight, which equals about 1ml Composition/kg subject body weight. In some embodiments, this could amount to about up to about 4mg lidocaine/kg subject weight, which equals about 0.08ml/kg of the Composition. In some embodiments, this could amount to about: cattle or other subjects (eg. from the bovine group) 500kg plus = up to about 40ml of a said Composition. In some embodiments, this could amount to about: calves or other subjects 20-500kg = up to about 12 ml of a said Composition. In some embodiments, this could amount to about: calves or other subjects 20-500kg = up to about 20 ml of a said Composition. In some embodiments, this could amount to about: cattle or other subjects 500kg plus = up to about 50ml or 60ml of a said Composition.

Preferred features, embodiments and variations of the invention may be discerned from the following Best Modes section which provides sufficient information for those skilled in the art to perform the invention, which is defined by the claims.

### Brief Description of Figures

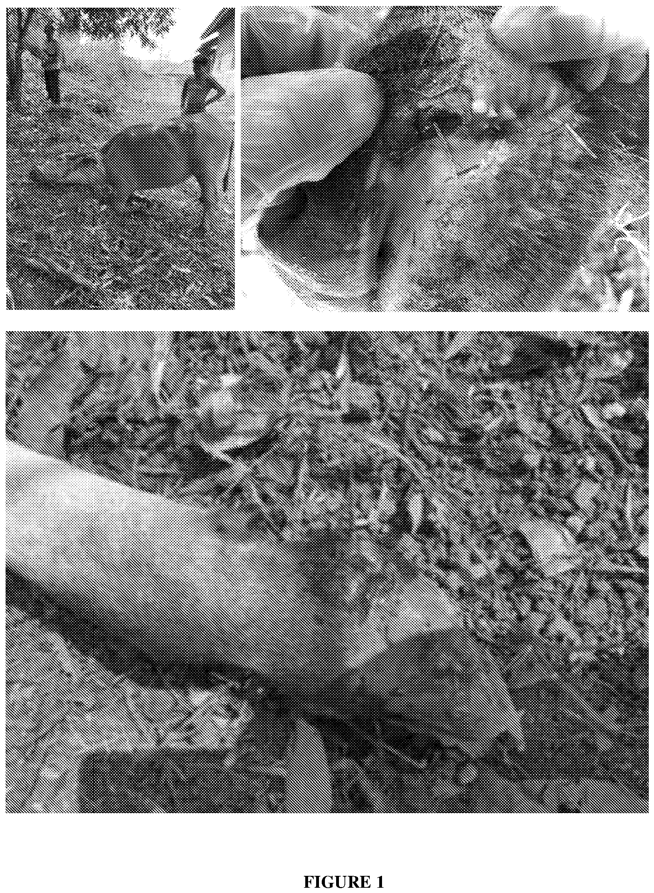

Figure 1 shows typical lesions (mouth and hoof) and body habitus (lameness) caused by FMD, prior to treatment with Tri-Solfen^{™} gel product.

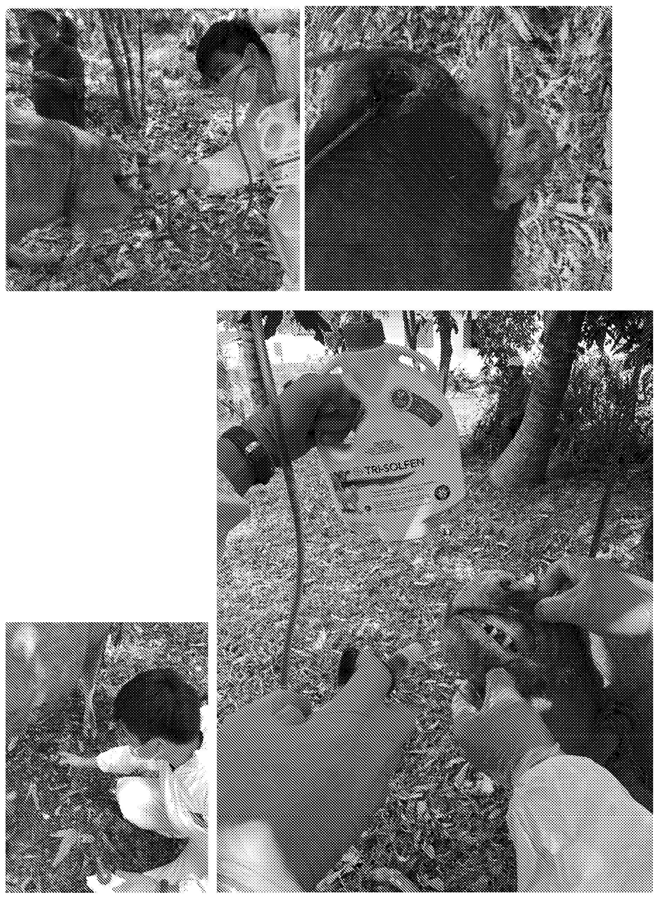

Figure 2 shows how the Tri-Solfen^{™} gel product was applied to animals suffering from FMD, using a liquid-gel applicator.

### Best Modes for Carrying Out the Invention

### Example 1 -Supervised Observational Field Trial of Tri-Solfen^{™} for Foot and Mouth Disease -Discovery of Anti-Viral Effects - Laos 2019

### Background

Foot and mouth-disease (FMD) is a highly infectious viral disease that affects animals with cloven hooves such as cattle, pigs, sheep, goats and artiodactyl wildlife species. The disease is characterized by fever, salivation and vesicles in the mouth, muzzle, dental pad, tongue, teats and feet. The rupture of the vesicles results in marked painful swelling of the coronary band, depression, innapetence, lameness, recumbency, loss of body condition, severe mastitis and abortions (Radostitis et al. 2000).

The global impact of foot and mouth disease (FMD) is colossal due to the huge numbers of animals affected. There are direct losses due to a reduction in production and changes in herd structure; and indirect losses that relate to the significant costs of FMD control and management and poor access to markets and limited use of improved production technologies. The annual impact of FMD in terms of production losses and vaccination alone are estimated as in the region of US$5 billion (Knight-Jones and Rushton 2013). Much of the global FMD burden of production losses falls on the world's poorest communities, particularly those most dependent on livestock in developing countries. The direct losses limit livestock productivity creating a food insecurity and contributing to malnutrition.

The FMD virus belongs to the *Aphthovirus* genus of *Picornaviridae* family, and its classification has seven serotypes (O, A, Asia 1, C, SAT 1, SAT 2, and SAT 3) and about 80 subtypes (Kahn et al. 2005). The serotypes have a large number of strains that have a spectrum of antigenic characteristics requiring more than one vaccine strain for each serotype (Kahn et al. 2005). There is no cross immunity between serotypes and this presents difficulties to vaccination programs (Radostitis et al. 2000).

The disease is highly contagious. The mode of transmission from infected animal to susceptible one is by inhalation, or direct contact with infected lesions or secretions. Exhaled air containing the virus infects susceptible animals via the respiratory or oral route. All body excretions and secretions from infected animal may contain the virus. Transmission to calves is via infected milk and fodder contaminated from contact with infected animals. Other sources of infection include: milk tankers, mechanical animal vectors like horses, cat, dogs, avian species and humans. Pigs are considered 'amplifying hosts'; often infected by contaminated feed derived from infected animals, spreading large volumes of virus that may spread the disease via aerosol to the cattle and other species (Kahn et al. 2005).

Disinfection during an outbreak can prevent the virus from spreading. The virus is known to be highly acid labile. FMD virus does not have an outer membrane and therefore is destroyed rapidly in conditions below pH 5.0 and above pH 11.0. At pH < 4 it undergoes rapid and complete destruction (Bachrach et al., 1957). Citric acid is commonly used for disinfection of animal housing and surrounding contaminated environments (Sellers et al., 1968).

FDM lesions are extremely painful for animals and results in lameness and pain on eating and drinking. Together these result in rapid loss of condition. Ulcers often take weeks to heal and foot lesions may become secondarily infected, compounding morbidity. There is no specific curative treatment for FMD. The conventional method of treating infected animals, where available, mainly involves the use of antibiotics, flunixin meglumine and mild disinfectants (Radostitis et al. 2000). In some countries FMD has been managed traditionally by use of natural soda ash solution for washing of the lesions and other communities have applied honey and even finger millet flour to the lesions (Gakuya et al., 2010). There are recent reports of improved healing following topical treatment of lesions with various agents including some antiseptics, phenytoin and lidocaine (Misk et al., 2015, Al-lethie et al., 2018).

Tri-Solfen^{™} (developed by Animal Ethics Pty Ltd) is a proprietary topical anaesthetic and antiseptic wound-care product that is applied directly to wounds in animals to mitigate pain, bleeding and infection. It contains two local anaesthetics; lidocaine/lignocaine, for rapid onset and bupivacaine, for prolonged duration of local anaesthetic effect, as well as adrenalin, to prevent systemic absorption of local anaesthetic actives, and cetrimide for antiseptic activity. Although not used to treat FMD cases until as described herein, Tri-Solfen^{™} is registered and widely used in livestock species (sheep and cattle) in Australia and New Zealand to mitigate pain due to wounds from surgical husbandry procedures such as castration, docking and dehorning and mulesing. It is applied directly to wounds using a "no touch" technique as a metered dose "spray and stay" formulation that adheres to the wound, providing a long-lasting coating over the wound to provide prolonged delivery of the actives. Numerous studies have reported significant reductions in pain associated with wounds in sheep, cattle and pigs (Lomax et al., 2008, 2010 and 2013, 2017). In cattle, as well as mitigating pain associated with castration, disbudding and dehorning wounds, it has also proven effective to mitigate pain associated with hoof lesions, resulting in improved management of bovine lameness (Stilwell et al., 2018).

### Observational Field Trial

A supervised observational trial of Tri-Solfen^{™} use for pain mitigation was performed during an FMD outbreak in buffalo in Laos in March 2019.

An outbreak of FMD in northern Laos was reported involving Ban Muangkhi, in Luang Prabang district of the Luang Prabang province. The outbreak involved 99 buffalo and 37 cows of a population of 194 buffalo and 44 cows, from 136 households. Clinical examination identified that the animals were affected with moderate to severe FMD lesion. All affected animals were treated with Tri-Solfen^{™} product (on oral and foot lesions). The Tri-Solfen^{™} product is a blue coloured liquid gel matrix that contains the following components: lidocaine hydrochloride; bupivacaine hydrochloride; adrenalin; and cetrimide. In particular, the Tri-Solfen^{™} commercial product contains:
Lidocaine hydrochloride 50g/L
Bupivacaine hydrochloride 5g/L
Adrenalin 1: 2000
Cetrimide 5g/L

Tri-Solfen^{™} product was applied using the "no-touch" metered-dose spray applicator directly to coat ulcerative lesions where-ever present in or around the mouth, nose or on the hooves. Applications were sufficient to coat the wounds without run-off, with total doses between 10-30ml applied per animal.

It was intended that in the affected animals, only a third of the pedal lesions would be treated with Tri-Solfen^{™}, a third would be treated with chlortetracycline, and third left untreated, to enable collection of data on the rate of healing. However, once the clinical response to Tri-Solfen^{™} product was observed, all farmers insisted that all lesions were to be treated 'with this new medicine that really works'.

### Results

On April 12th, we requested that all interested farmers with FMD affected animals that agreed to trial the 'new medicine', assemble their affected animals for examination, collection of samples and potentially treatment of the FMD lesions with the wound therapy formulation. Following the initial treatments and the observations of very positive clinical effects, all the farmers (n=15) in the village with FMD-affected buffalo (n=99) and cattle (n=37) presented their animals for treatment. This was from a village population of 194 buffalo and 44 cows and involved only 15 of 136 households. The lower rate of households impacted with FMD than expected, was considered attributable to FMD vaccination that had been conducted in the village 5 months previously. Although present in the village, no goats or pigs were reported to have been affected by FMD.

On arrival at the village, the initial observations were that the normally quiet and passive animals were hyperaesthetic and agitated, reluctant to move, with several preferring recumbency to being restrained by the 'bleeding pole' method commonly used in developing countries where cattle crushes are largely absent. Although both FMD-vaccinated and unvaccinated buffalo and cattle were present in the village, clinical signs were only observed in the unvaccinated animals.

Clinical examinations confirmed the presence of lesions of severe, subacute, ulcerative glossitis extending to involve the nasal muvosa, plus moderate subacute ulcerative interdigital dermatitis. These lesions were considered typical of FMD of a few days' duration. Oral ulcerations were between 5 and 15cm in diameter. Pedal lesions were most commonly located in interdigital issue but were occasionally spread across the coronary band and were between 5 and 10cm in diameter. Following aseptic collection of necrotic oral mucosal tissue from 10 animals, between 10-30 millilitres of Tri-Solfen^{™} product per animal was sprayed directly onto the oral and feet lesions (no teat lesions were observed). This resulted in immediate improvement in demeanour and locomotion of the animals. No adverse events were observed or reported.

The clinical impacts of treatment of a large number of FMD-affected large ruminants (n=136) with the pain-relief topical anaesthetic wound formulation Tri-Solfen^{™}, were readily observable by all present on April 12th. Further, the follow-up interviews with the farmers also indicated that the expected response and recovery from the disease was considered markedly improved from known disease progression and recovery times for FMD-affected animals following use of other treatments.

On interview a week later, all 15 farmers advised that their animals were eating within 2 days and lesions had recovered within 5 days. They all also advised they were keen to purchase the product for future use. Farmers described that the treatment had clearly provided a rapid improvement in behaviour indicative of a dramatic reduction in pain, as well as an improvement in healing rates, with animals eating within 2 days and lesions having recovered within 3-5 days. Although the laboratory reported that the tissue samples were unsuitable for antigen testing or submission for virus isolation, samples collected from a previous outbreak in a nearby village identified the FMD involved in recent outbreaks as serotype O (Panasian topotype).

As a result of these findings, the Lao veterinary chemical authority rapidly registered the product for the purpose of treating FMD. At the time of writing, a similar positive episode of treatment with this new therapy had recently occurred, involving a large outbreak of FMD in the adjacent province in Laos (Huaphan).

Applications were extremely well tolerated by animals and noted to result in a rapid improvement in habitus, locomotion, and behaviour. There were no adverse events observed on application or reported during follow-up interviews.

Farmers described that treatment provided a rapid improvement in behaviour indicative of a dramatic reduction in pain, as well as a rapid improvement in healing, with animals able to eat within 2 days and lesions having recovered within 3-5 days.

These were considered markedly improved from known disease progression and recovery times for FMD-affected animals.

### Discussion

The pain-alleviation effects of Tri-Solfen^{™} product observed in this trial, along with absence of adverse effects, are highly consistent with positive pain relieving effects reported in cattle with wounds from other sources as detailed above. The flow-on effects in buffalo/cattle with FMD have resulted in markedly improved locomotion feeding and recovery, with dramatic potential benefits for health and welfare of cattle/buffalo and communities in FMD outbreaks.

Furthermore, a dramatic effect on the speed of resolution of the FMD lesions has been observed. This is a novel discovery indicative of likely direct anti-viral activity of Tri-Solfen^{™} product against FMD virus. This anti-viral activity should limit virus transmission during outbreaks. Further, the positive clinical impacts encourage farmers to seek treatment, increasing the proportion of the affected population that is administered an antiviral medication. These positive impacts are likely to influence the spread of the disease, minimising the extent of suffering and economic losses.

Direct anti-viral activity of Tri-Solfen^{™} product is thought to proceed from a unique synergistic combination of factors. As noted, FMD virus has high sensitivity to acid environments and is rapidly destroyed at pH <4. Tri-Solfen^{™} product has a pH of 2.7 and thus has direct viricidal activity against FMD virus when applied to exposed lesions where virus is still present. In general, the application of acidic solutions to open wounds and ulcers, such as present in FMD, is contraindicated as the acidity may exacerbate pain and be poorly tolerated. In the case of Tri-Solfen^{™} product however the relatively high concentration of lidocaine (5%) applied with adrenalin is known to result in rapid and prolonged wound anaesthesia. This combination therefore, uniquely delivers to the lesion, a long-lasting solution with acidity sufficient to destroy the virus, without causing pain to the animal.

Furthermore, lidocaine, at concentrations present in Tri-Solfen^{™} product, should have direct viricidal effects. Although it has not been tested for activity against FMD until now, lidocaine and its pharmaceutically active salts are known to exhibit antiviral activity against Herpes Virus in cell culture and animal model systems at concentrations ranging from 0.5mg/ml (0.05%) to 100mg/ml (10%) (Haines et al., 1986). Cetrimide, another active in Tri-Solfen^{™} product, is a quaternary ammonium antiseptic also with known antiviral activity, however may or may not have activity against FMD virus, as quaternary ammonium compounds are generally considered ineffective against viruses without lipid membranes (such as FMD) (Shirai 2012).

The combined effect of the acidic pH, with the antiseptic and antiviral effects of the actives appears to have eradicated the virus from exposed lesions and associated inflammatory secretions, as well as minimising secondary bacterial infection. This, in turn, may not only reduce viral and bacterial load on the animal (and hence hasten healing and recovery) but should also eliminate viral shedding from wounds, and prevent spread of the disease to other animals or the environment via contact with infected lesions or inflammatory secretions from wounds.

Such a combination of effects may be anticipated to deliver profound benefits for the well-being of animals, herds and communities affected by FMD outbreaks. The potential to not only reduce animal suffering and speed recovery but also to greatly reduce the extent and severity of outbreaks may have profound health, welfare and economic benefits.

Since this outbreak in Laos in April 2019, clinical trials with Tri-Solfen^{™} product have been conducted in several countries in Africa, with all reports indicating very high level (100%) acceptance by farmers, of the application of this wound pain-relief therapeutic compound to FMD lesions.

### Example 2 - Effect of a topical anaesthetic formulation on viral load in lambs naturally infected with orf virus

### Objectives

Orf is a highly contagious eruptive skin condition of sheep and goats, caused by a Parapoxvirus with a worldwide distribution. It affects mainly lambs and kids, with more serious outbreaks often associated with intensive husbandry, causing significant financial losses to livestock production. It is also a zoonotic disease, affecting mainly people via direct or indirect contact with infected animals. Vaccination remains the preferred option to control the disease. However, currently in Spain and many other countries, no orf vaccine is available. The treatment of this disorder referred to as Contagious Ecthyma and Scabby Mouth, involves standard hygiene practices and management of presumptive secondary infections.

### Materials and methods

Fourteen one-month-old Rasa Aragonesa lambs, naturally infected with orf, were recruited from a farm where an outbreak of orf disease was occurring. The animals were divided into two cohorts: Group A (n=11) consisting of animals with orf lesions treated with Tri-Solfen^{™} product and Group B (n=3), a control group without treatment.

Cotton swabs were obtained before treatment (T0) and days 1 (T1), 3 (T2) and 5 (T3) post-treatment, then submitted to direct DNA extraction and real-time PCR quantification (Exopol) or to incubation with primary tissue cultures from ovine skin fibroblasts (OSF) and T-immortalized goat embryonic fibroblasts (TIGEF). Orf quantification was performed by real time PCR on DNA from cultured cells at day 0 and 5 post-treatment. Data were analyzed using the non-parametric Wilcoxon test for paired samples and by T-Student's test for unrelated samples.

### Results

In the study carried out using quantitative PCR, no significant differences were found between day 0 pre-treatment (T0) and day 5 post-treatment (T3) (p=0.722). However, when the viral load was assessed in primary tissue cultures of ovine skin fibroblasts (OSF) and T-immortalized goat embryonic fibroblasts (TIGEF), there was a reduction in both groups between T0 and T3 that was significant in the OSF cell cultures (p<0.05).

### Conclusions

These results suggest that despite the presence of the viral DNA in the orf lesions at 5 days post-treatment, this may belong to inactivated virus as the viral load obtained after cell culture of the samples of the treated animals was significantly less than that obtained from controls. These findings suggest that as treatment of orf lesions with Tri-Solfen^{™} product reduces the viral load present in lesions, such therapy may also alter the clinical progression and transmission in outbreaks of Contagious Ecthyma.

### Example 3 - Use of Tri-Solfen^{™} Product for Other Pathogenic Diseases

The inventors have made a surprising discovery that the Tri-Solfen^{™} gel product is particularly effect in treating and controlling FMD in bovine species when applied to a diseased area of the animal. In view of the surprising efficacy of the Tri-Solfen^{™} product, the inventors postulate that similar compositions having both viricidal and rapidly acting pain-relieving properties can be used to treat and control FMD - especially compositions that are adhesive/sticky in relatively dry diseased areas and/or foam in relatively wet diseased areas. The inventors also postulate that the Tri-Solfen^{™} product and like products can be used to treat and control other foot or mouth-type pathogenic diseases, including scabby mouth and hoof rot/footrot/foot abscess.

The inventors also postulate that the Tri-Solfen^{™} product and like products can be used to treat and control other viral, bacterial or fungal diseases in humans and animals - particularly those that are acid-labile, such as Herpes, Parvovirus, Rhino-virus and Equine rhinitis A virus, and Candida and Tinea.

The inventors further postulate that the Tri-Solfen^{™} product and like products can be used to treat and control diseases in humans and animals caused by infestational pathogens, such as those causing flystrike - particularly those that are acid-labile, such as screw worm and blowfly larvae.

Treatment of diseased animals or humans could be carried out, largely as described for FMD, by applying the composition/solution topically to all exposed lesions (denuded, excoriated, inflamed, blistered, erupted or ulcerated skin or mucous membranes) in sufficient amount to coat the surface of the lesions.

Throughout this specification, unless in the context of usage an alternative interpretation is required, the term "comprise" (and variants thereof such as "comprising" and "comprised") denotes the inclusion of a stated integer or integers but does not exclude the presence of another integer or other integers.

Any reference to publications cited in this specification is not an admission that the disclosures constitute common general knowledge in Australia or in other countries.

### References

Radostitis OM, Gay CC, Blood DC, Hinchcliff KW. Veterinary medicine: A textbook of the diseases of cattle, sheep, pigs, goats and horses. Ninth edition. London. UK: W.B. Saunders company. Harcourt publishers; 2000. pp. 1059-1066.
Knight-Jones, T.J.D., and J. Rushton. "The Economic Impacts of Foot and Mouth Disease - What Are They, How Big Are They and Where Do They Occur?" Preventive Veterinary Medicine 112, no. 3-4 (November 1, 2013): 161-73. https://doi.org/10.1016/j.prevetmed.2013.07.013.
Kahn CN, Line S. The Mercks Veterinary Manual. Ninth Edition. New Jersey, USA: Merck and Company Incorporated, White House Station; 2005. pp. 507-510.
Bachrach, H. L., S. S. Breese, J. J. Callis, W. R. Hess, and R. E. Patty. "Inactivation of Foot-and-Mouth Disease Virus by PH and Temperature Changes and by Formaldehyde." Proceedings of the Society for Experimental Biology and Medicine 95, no. 1 (May 1, 1957): 147-52. https://doi.org/10.3181/00379727-95-23148.
Sellers, R. F. "The Inactivation of Foot-and Mouth Disease Virus by Chemicals and Disinfectants." The Veterinary Record 83, no. 20 (November 16, 1968): 504-6.
Gakuya, DW, CM Mulei, and S B Wekesa. "Use of Ethnoveterinary Remedies in the Management of Foot and Mouth Disease Lesions in a Diary Herd." African Journal of Traditional, Complementary, and Alternative Medicines 8, no. 2 (December 30, 2010): 165-69. https://www.ncbi.nlm.nih.gov/pmc/articles/PMC3252696/.
Misk, Nabil, Tarik Misk, and H Z Rateb. "Assessment and topical treatment of lesions of foot and mouth disease in cattle." Assiut Vet. Med. J. Vol. 61 No. 145, April 1, 2015.
Al-lethie, Al-Lethie, Sayed F El-Hawari, Khaled El-Khabaz, Enas Elmeligy, Arafat Khalphallah, and Usama Mahmoud. "Evaluation of clinical recovery and healing of oral lesions by 3 different therapeutic regimens in cattle with foot and mouth disease (FMD)" 64 (January 1, 2018).
Lomax, S, M Sheil, and P A Windsor. "Impact of Topical Anaesthesia on Pain Alleviation and Wound Healing in Lambs after Mulesing." Australian Veterinary Journal 86, no. 5 (May 2008): 159-68. https://doi.org/10.1111/j.1751-0813.2008.00285.x.
Lomax, S., and P. A. Windsor. "Topical Anesthesia Mitigates the Pain of Castration in Beef Calves." Journal of Animal Science 91, no. 10 (October 1, 2013): 4945-52. https://doi.org/10.2527/jas.2012-5984.
Lomax, S, M Sheil, and Pa Windsor. "Duration of Action of a Topical Anaesthetic Formulation for Pain Management of Mulesing in Sheep." Australian Veterinary Journal 91, no. 4 (April 2013): 160-67. https://doi.org/10.1111/avj.12031.
Lomax, S, H Dickson, M Sheil, and P A Windsor. "Topical Anaesthesia Alleviates Short-Term Pain of Castration and Tail Docking in Lambs." Australian Veterinary Journal 88, no. 3 (March 2010): 67-74. https://doi.org/10.1111/j.1751-0813.2009.00546.x.
Lomax, Sabrina, Charissa Harris, Peter A. Windsor, and Peter J. White. "Topical Anaesthesia Reduces Sensitivity of Castration Wounds in Neonatal Piglets." PloS One 12, no. 11 (2017): e0187988.
Stilwell, G.T., A.M. Ferrador, M.S. Santos, J.M. Domingues, and N. Carolino. "Use of Topical Local Anesthetics to Control Pain during Treatment of Hoof Lesions in Dairy Cows." Journal of Dairy Science, April 2019. https://doi.org/10.3168/jds.2018-15820.
Haines et al. Antiviral pharmaceutical preparations and methods for their use- United States Patent Number: 4,628,063 45: Dec.9,1986.
Shirai, Junsuke. "Disinfection Against the Outbreaks of Foot and Mouth Disease (FMD)." Journal of Disaster Research 7, no. 3 (April 1, 2012): 264-73. https://doi.org/10.20965/jdr.2012.p0264.

## Claims

1. A topical composition for use in the treatment of a pathogenic disease caused by an acid-labile pathogen selected from foot and mouth disease (FMD), scabby mouth (Orf), Picornavirus, Parapoxvirus, and a pathogenic disease causing pox lesions, **characterised in that**:
the composition has an acidic pH;
the composition is applied to a subject at therapeutically effective amounts; and
the composition comprises:
(a) Composition 1 comprising:
about 100 mg/ml non-crystallising liquid sorbitol (70%);
about 50.0 mg/ml lignocaine HCl;
about 5.0 mg/ml bupivacaine HCl;
about 1.5 mg/ml sodium metabisulfite;
about 5.0 mg/ml cetrimide;
about 45.0 µg/ml adrenaline tartrate;
about 5.0 mg/ml hydroxy cellulose; and optionally
colourant;
(b) Composition 2 whereby lignocaine of Composition 1 is replaced by tetracaine at about 10 mg/ml - 100 mg/ml;
(c) Composition 3 comprising:
about 100 mg/ml non-crystallising liquid sorbitol (70%);
about 40.0 mg/ml lignocaine HCl;
about 1.5 mg/ml sodium metabisulfite;
about 5.0 mg/ml cetrimide;
about 36.0 µg/ml adrenaline tartrate;
about 5.0 mg/ml hydroxy cellulose; and optionally
colourant;
(d) Composition 4 whereby lignocaine of Composition 3 is replaced by tetracaine at about 10 mg/ml - 100 mg/ml;
(e) Composition 5 comprising:
about 100.0 mg/ml purified water sorbitol liquid 70% non-crystallising;
about 50.0 mg/ml (5%) tetracaine HCl;
about 1.5 mg/ml sodium metabisulfite;
about 5.0 mg/ml cetrimide;
about 45.0 µg/ml adrenaline tartrate;
about 5.0 mg/ml hydroxy cellulose;
to 1 ml purified water; and optionally
colourant (quantity to suit);
(f) Composition 6 comprising: lignocaine, bupivacaine, adrenaline, cetrimide, 2-hydroxyethyl cellulose, sodium metabisulfite, liquid sorbitol (70%), buffer, and, optionally colourant;
(g) Composition 7 whereby lignocaine of Composition 6 is replaced by tetracaine at about 10 mg/ml - 100 mg/ml;
(h) Composition 8 comprising: amethocaine / tetracaine, adrenaline, cetrimide, 2-hydroxyethyl cellulose, sodium metabisulfite, liquid sorbitol (70%), buffer, and, optionally, colourant;
(i) Composition 9 comprising a liquid gel matrix that contains the following:
lidocaine;
adrenalin; and
cetrimide,
and the composition is optionally coloured, wherein said composition has a pH lower than about 4.0;
(j) Composition 10 comprising a liquid gel matrix that contains the following: tetracaine; adrenalin; and cetrimide, and the composition is optionally coloured, wherein said composition has a pH lower than about 4.0; or
(k) Composition 11 comprising a liquid gel matrix that contains the following: lidocaine; bupivacaine; adrenalin; and cetrimide, and the composition is optionally coloured, wherein said composition has a pH lower than about 4.0.

2. A topical composition for use in the treatment of a diseased area of a subject caused by or aggravated by an acid-labile pathogen selected from the group consisting of foot and mouth disease (FMD), scabby mouth (Orf), Picornavirus, Parapoxvirus, and a pathogenic disease causing pox lesions, **characterised in that**:
the composition has an acidic pH;
the composition is applied to the diseased area; and
the composition comprises:
(a) Composition 1 comprising:
about 100 mg/ml non-crystallising liquid sorbitol (70%);
about 50.0 mg/ml lignocaine HCl;
about 5.0 mg/ml bupivacaine HCl;
about 1.5 mg/ml sodium metabisulfite;
about 5.0 mg/ml cetrimide;
about 45.0 µg/ml adrenaline tartrate;
about 5.0 mg/ml hydroxy cellulose; and optionally
colourant;
(b) Composition 2 whereby lignocaine of Composition 1 is replaced by tetracaine at about 10 mg/ml - 100 mg/ml;
(c) Composition 3 comprising:
about 100 mg/ml non-crystallising liquid sorbitol (70%);
about 40.0 mg/ml lignocaine HCl;
about 1.5 mg/ml sodium metabisulfite;
about 5.0 mg/ml cetrimide;
about 36.0 µg/ml adrenaline tartrate;
about 5.0 mg/ml hydroxy cellulose; and optionally
colourant;
(d) Composition 4 whereby lignocaine of Composition 3 is replaced by tetracaine at about 10 mg/ml - 100 mg/ml;
(e) Composition 5 comprising:
about 100.0 mg/ml purified water sorbitol liquid 70% non-crystallising;
about 50.0 mg/ml (5%) tetracaine HCl;
about 1.5 mg/ml sodium metabisulfite;
about 5.0 mg/ml cetrimide;
about 45.0 µg/ml adrenaline tartrate;
about 5.0 mg/ml hydroxy cellulose;
to 1 ml purified water; and optionally
colourant (quantity to suit);
(f) Composition 6 comprising: lignocaine, bupivacaine, adrenaline, cetrimide, 2-hydroxyethyl cellulose, sodium metabisulfite, liquid sorbitol (70%), buffer, and, optionally colourant;
(g) Composition 7 whereby lignocaine of Composition 6 is replaced by tetracaine at about 10 mg/ml - 100 mg/ml;
(h) Composition 8 comprising: amethocaine / tetracaine, adrenaline, cetrimide, 2-hydroxyethyl cellulose, sodium metabisulfite, liquid sorbitol (70%), buffer, and, optionally, colourant;
(i) Composition 9 comprising a liquid gel matrix that contains the following:
lidocaine;
adrenalin; and
cetrimide,
and the composition is optionally coloured, wherein said composition has a pH lower than about 4.0;
(j) Composition 10 comprising a liquid gel matrix that contains the following: tetracaine; adrenalin; and cetrimide, and the composition is optionally coloured, wherein said composition has a pH lower than about 4.0; or
(k) Composition 11 comprising a liquid gel matrix that contains the following: lidocaine; bupivacaine; adrenalin; and cetrimide, and the composition is optionally coloured, wherein said composition has a pH lower than about 4.0.

3. The topical composition for use according to claim 1 or claim 2, wherein the pathogenic disease is scabby mouth (Orf).

4. The topical composition for use according to claim 1 or claim 2, wherein the pathogenic disease is foot and mouth disease (FMD).

## Patentansprüche

1. Topische Zusammensetzung zur Verwendung bei der Behandlung einer pathogenen Erkrankung, die durch einen säurelabilen Erreger verursacht wird, der aus Maul- und Klauenseuche (MKS), ansteckendem Ecthyma (Orf), Picornavirus, Parapoxvirus und einer pathogenen Erkrankung, die papulöse Läsionen verursacht, ausgewählt ist, **dadurch gekennzeichnet, dass** die Zusammensetzung einen sauren pH-Wert aufweist;
die Zusammensetzung in therapeutisch wirksamen Mengen auf ein Subjekt aufgetragen wird; und die Zusammensetzung Folgendes umfasst:
(a) eine Zusammensetzung 1, die Folgendes umfasst:
ca. 100 mg/ml nicht kristallisierendes flüssiges Sorbitol (70 %); ca. 50,0 mg/ml Lignocainhydrochlorid;
ca. 5,0 mg/ml Bupivacainhydrochlorid;
ca. 1,5 mg/ml Natriummetabisulfit;
ca. 5,0 mg/ml Cetrimid;
ca. 45,0 µg/ml Adrenalintartrat;
ca. 5,0 mg/ml Hydroxycellulose; und gegebenenfalls einen Farbstoff;
(b) eine Zusammensetzung 2, in der das Lignocain in der Zusammensetzung 1 durch Tetracain in einer Menge von etwa 10 mg/ml - 100 mg/ml ersetzt ist;
(c) eine Zusammensetzung 3, die Folgendes umfasst:
ca. 100 mg/ml nicht kristallisierendes flüssiges Sorbitol (70 %);
ca. 40,0 mg/ml Lignocainhydrochlorid;
ca. 1,5 mg/ml Natriummetabisulfit;
ca. 5,0 mg/ml Cetrimid;
ca. 36,0 µg/ml Adrenalintartrat;
ca. 5,0 mg/ml Hydroxycellulose; und gegebenenfalls einen Farbstoff;
(d) eine Zusammensetzung 4, in der das Lignocain in der Zusammensetzung 3 durch Tetracain in einer Menge von etwa 10 mg/ml - 100 mg/ml ersetzt ist;
(e) eine Zusammensetzung 5, die Folgendes umfasst:
ca. 100,0 mg/ml gereinigtes Wasser und 70 % flüssiges, nicht kristallisierendes Sorbitol;
ca. 50,0 mg/ml (5 %) Tetracainhydrochlorid;
ca. 1,5 mg/ml Natriummetabisulfit;
ca. 5,0 mg/ml Cetrimid;
ca. 45,0 µg/ml Adrenalintartrat;
ca. 5,0 mg/ml Hydroxycellulose;
auf 1 ml gereinigtes Wasser; und gegebenenfalls einen Farbstoff (Menge anpassen);
(f) eine Zusammensetzung 6, die Folgendes umfasst: Lignocain, Bupivacain, Adrenalin, Cetrimid, 2-Hydroxyethylcellulose, Natriummetabisulfit, flüssiges Sorbitol (70 %), eine Pufferlösung und gegebenenfalls einen Farbstoff;
(g) eine Zusammensetzung 7, in der das Lignocain in der Zusammensetzung 6 durch Tetracain in einer Menge von etwa 10 mg/ml - 100 mg/ml ersetzt ist;
(h) eine Zusammensetzung 8, die folgendes umfasst: Amethocain/Tetracain, Adrenalin, Cetrimid, 2-Hydroxyethylcellulose, Natriummetabisulfit, flüssigem Sorbitol (70 %), eine Pufferlösung und gegebenenfalls einen Farbstoff ;
(i) eine Zusammensetzung 9, umfassend eine flüssige Gelmatrix, die Folgendes enthält:
Lidocain;
Adrenalin; und
Cetrimid,
wobei die Zusammensetzung gegebenenfalls gefärbt ist, und in der die besagte Zusammensetzung einen pH-Wert von weniger als ca. 4,0 aufweist;
(j) eine Zusammensetzung 10, umfassend eine flüssige Gelmatrix, die die folgenden Elemente enthält: Tetracain, Adrenalin und Cetrimid, wobei die Zusammensetzung gegebenenfalls gefärbt ist, und in der die besagte Zusammensetzung einen pH-Wert von weniger als ca. 4,0 aufweist; oder
(k) eine Zusammensetzung 11, umfassend eine flüssige Gelmatrix, die die folgenden Elemente enthält: Lidocain, Bupivacain, Adrenalin und Cetrimid, wobei die Zusammensetzung gegebenenfalls gefärbt ist, und in der die besagte Zusammensetzung einen pH-Wert von weniger als ca. 4,0 aufweist.

2. Topische Zusammensetzung zur Verwendung bei der Behandlung eines erkrankten Bereichs eines Subjekts, der durch einen säurelabilen Krankheitserreger verursacht oder verschlimmert wird, der aus der Gruppe ausgewählt ist, die aus Maul- und Klauenseuche (MKS), Ecthyma contagiosum (Orf), Picornavirus, Parapoxvirus und einer pathogenen Krankheit besteht, die papulöse Läsionen verursacht, **dadurch gekennzeichnet, dass** :
die Zusammensetzung einen sauren pH-Wert hat;
die Zusammensetzung auf den erkrankten Bereich aufgetragen wird; und
die Zusammensetzung Folgendes umfasst:
(a) eine Zusammensetzung 1, umfassend:
ca. 100 mg/ml nicht kristallisierendes flüssiges Sorbitol (70 %);
ca. 50,0 mg/ml Lignocainhydrochlorid;
ca. 5,0 mg/ml Bupivacainhydrochlorid;
ca. 1,5 mg/ml Natriummetabisulfit;
ca. 5,0 mg/ml Cetrimid;
ca. 45,0 µg/ml Adrenalintartrat;
ca. 5,0 mg/ml Hydroxycellulose; und gegebenenfalls einen Farbstoff;
(b) eine Zusammensetzung 2, in der das Lignocain in Zusammensetzung 1 durch Tetracain in einer Menge von etwa 10 mg/ml - 100 mg/ml ersetzt ist;
(c) eine Zusammensetzung 3, die Folgendes umfasst:
ca. 100 mg/ml nicht kristallisierendem flüssigen Sorbitol (70 %);
ca. 40,0 mg/ml Lignocainhydrochlorid;
ca. 1,5 mg/ml Natriummetabisulfit;
ca. 5,0 mg/ml Cetrimid;
ca. 36,0 µg/ml Adrenalintartrat;
ca. 5,0 mg/ml Hydroxycellulose; und gegebenenfalls einen Farbstoff;
(d) eine Zusammensetzung 4, in der das Lignocain in Zusammensetzung 3 durch Tetracain in einer Menge von etwa 10 mg/ml - 100 mg/ml ersetzt ist;
(e) eine Zusammensetzung 5, die Folgendes umfasst:
ca. 100,0 mg/ml gereinigtes Wasser und 70 % flüssiges, nicht kristallisierendes Sorbitol;
ca. 50,0 mg/ml (5 %) Tetracainhydrochlorid;
ca. 1,5 mg/ml Natriummetabisulfit;
ca. 5,0 mg/ml Cetrimid;
ca. 45,0 µg/ml Adrenalintartrat;
ca. 5,0 mg/ml Hydroxycellulose;
auf 1 ml gereinigtes Wasser; und gegebenenfalls einen Farbstoff (Menge anpassen);
(f) eine Zusammensetzung 6, die Folgendes umfasst: Lignocain, Bupivacain, Adrenalin, Cetrimid, 2-Hydroxyethylcellulose, Natriummetabisulfit, flüssiges Sorbitol (70 %), eine Pufferlösung und gegebenenfalls einen Farbstoff;
(g) eine Zusammensetzung 7, in der das Lignocain in Zusammensetzung 6 durch Tetracain in einer Menge von etwa 10 mg/ml - 100 mg/ml ersetzt ist;
(h) eine Zusammensetzung 8, die folgendes umfasst: Amethocain/Tetracain, Adrenalin, Cetrimid, 2-Hydroxyethylcellulose, Natriummetabisulfit, flüssigem Sorbitol (70 %), eine Pufferlösung und gegebenenfalls einen Farbstoff ;
(i) eine Zusammensetzung 9, umfassend eine flüssige Gelmatrix, die die folgenden Elemente enthält:
Lidocain; Adrenalin; und
Cetrimid,
wobei die Zusammensetzung gegebenenfalls gefärbt ist, und in der die besagte Zusammensetzung einen pH-Wert von weniger als ca. 4,0 aufweist;
(j) eine Zusammensetzung 10, umfassend eine flüssige Gelmatrix, die die folgenden Elemente enthält: Tetracain, Adrenalin und Cetrimid, wobei die Zusammensetzung gegebenenfalls gefärbt ist, und in der die besagte Zusammensetzung einen pH-Wert von weniger als ca. 4,0 aufweist; oder
(k) eine Zusammensetzung 11, umfassend eine flüssige Gelmatrix, die die folgenden Elemente enthält: Lidocain, Bupivacain, Adrenalin und Cetrimid, wobei die Zusammensetzung gegebenenfalls gefärbt ist, und in der die besagte Zusammensetzung einen pH-Wert von weniger als ca. 4,0 aufweist.

3. Topische Zusammensetzung zur Verwendung nach Anspruch 1 oder Anspruch 2, wobei die pathogene Erkrankung Ecthyma contagiosum (Orf) ist.

4. Topische Zusammensetzung zur Verwendung nach Anspruch 1 oder 2, wobei die pathogene Erkrankung die Maul- und Klauenseuche (MKS) ist.

## Revendications

1. Composition topique destinée à être utilisée dans le traitement d'une maladie pathogène causée par un agent pathogène acido-labile choisi dans la fièvre aphteuse (FMD), l'ecthyma contagieux (Orf), les picornavirus, les parapoxvirus et une maladie pathogène causant des lésions papuleuses, **caractérisée en ce que** : la composition a un pH acide ;
la composition est appliquée à un sujet dans des quantités thérapeutiquement efficaces ; et la composition comprend :
(a) Composition 1 comprenant :
environ 100 mg/ml de sorbitol liquide non cristallisant (70 %) ; environ 50,0 mg/ml de chlorhydrate de lignocaïne ;
environ 5,0 mg/ml de chlorhydrate de bupivacaïne ;
environ 1,5 mg/ml de métabisulfite de sodium ;
environ 5,0 mg/ml de cétrimide ;
environ 45,0 µg/ml de tartrate d'adrénaline ;
environ 5,0 mg/ml d'hydroxycellulose ; et éventuellement un colorant ;
(b) Composition 2 dans laquelle la lignocaïne de la Composition 1 est remplacée par de la tétracaïne à raison d'environ 10 mg/ml - 100 mg/ml ;
(c) Composition 3 comprenant :
environ 100 mg/ml de sorbitol liquide non cristallisant (70 %) ;
environ 40,0 mg/ml de chlorhydrate de lignocaïne ;
environ 1,5 mg/ml de métabisulfite de sodium ;
environ 5,0 mg/ml de cétrimide ;
environ 36,0 µg/ml de tartrate d'adrénaline ;
environ 5,0 mg/ml d'hydroxycellulose ; et éventuellement un colorant ;
(d) Composition 4 dans laquelle la lignocaïne de la Composition 3 est remplacée par de la tétracaïne à raison d'environ 10 mg/ml - 100 mg/ml ;
(e) Composition 5 comprenant :
environ 100,0 mg/ml d'eau purifiée et sorbitol liquide 70 % non cristallisant ;
environ 50,0 mg/ml (5 %) de chlorhydrate de tétracaïne ;
environ 1,5 mg/ml de métabisulfite de sodium ;
environ 5,0 mg/ml de cétrimide ;
environ 45,0 µg/ml de tartrate d'adrénaline ;
environ 5,0 mg/ml d'hydroxycellulose ;
à 1 ml d'eau purifiée ; et éventuellement un colorant (quantité à adapter) ;
(f) Composition 6 comprenant : lignocaïne, bupivacaïne, adrénaline, cétrimide, 2-hydroxyéthylcellulose, métabisulfite de sodium, sorbitol liquide (70 %), tampon et, éventuellement, colorant ;
(g) Composition 7 dans laquelle la lignocaïne de la Composition 6 est remplacée par de la tétracaïne à raison d'environ 10 mg/ml - 100 mg/ml ;
(h) Composition 8 comprenant : améthocaïne / tétracaïne, adrénaline, cétrimide, 2-hydroxyéthylcellulose, métabisulfite de sodium, sorbitol liquide (70 %), tampon et, éventuellement, colorant ;
(i) Composition 9 comprenant une matrice de gel liquide contenant les éléments suivants :
lidocaïne ;
adrénaline ; et
cétrimide,
et la composition est éventuellement colorée, dans laquelle ladite composition a un pH inférieur à environ 4,0;
(j) Composition 10 comprenant une matrice de gel liquide qui contient les éléments suivants : tétracaïne, adrénaline et cétrimide, et la composition est éventuellement colorée, dans laquelle ladite composition a un pH inférieur à environ 4,0; ou
(k) Composition 11 comprenant une matrice de gel liquide qui contient les éléments suivants : lidocaïne, bupivacaïne, adrénaline et cétrimide, et la composition est éventuellement colorée, dans laquelle ladite composition a un pH inférieur à environ 4,0.

2. Composition topique destinée à être utilisée dans le traitement d'une zone malade d'un sujet, causée ou aggravée par un agent pathogène acido-labile choisi dans le groupe consistant en fièvre aphteuse (FMD), ecthyma contagieux (Orf), picornavirus, parapoxvirus et une maladie pathogène causant des lésions papuleuses, **caractérisée en ce que** :
la composition a un pH acide ;
la composition est appliquée sur la zone malade ; et
la composition comprend :
(a) Composition 1 comprenant :
environ 100 mg/ml de sorbitol liquide non cristallisant (70 %) ;
environ 50,0 mg/ml de chlorhydrate de lignocaïne ;
environ 5,0 mg/ml de chlorhydrate de bupivacaïne ;
environ 1,5 mg/ml de métabisulfite de sodium ;
environ 5,0 mg/ml de cétrimide ;
environ 45,0 µg/ml de tartrate d'adrénaline ;
environ 5,0 mg/ml d'hydroxycellulose ; et éventuellement un colorant ;
(b) Composition 2 dans laquelle la lignocaïne de la Composition 1 est remplacée par de la tétracaïne à raison d'environ 10 mg/ml - 100 mg/ml ;
(c) Composition 3 comprenant :
environ 100 mg/ml de sorbitol liquide non cristallisant (70 %) ;
environ 40,0 mg/ml de chlorhydrate de lignocaïne ;
environ 1,5 mg/ml de métabisulfite de sodium ;
environ 5,0 mg/ml de cétrimide ;
environ 36,0 µg/ml de tartrate d'adrénaline ;
environ 5,0 mg/ml d'hydroxycellulose ; et éventuellement un colorant ;
(d) Composition 4 dans laquelle la lignocaïne de la Composition 3 est remplacée par de la tétracaïne à raison d'environ 10 mg/ml - 100 mg/ml ;
(e) Composition 5 comprenant :
environ 100,0 mg/ml d'eau purifiée et sorbitol liquide 70 % non cristallisant ;
environ 50,0 mg/ml (5 %) de chlorhydrate de tétracaïne ;
environ 1,5 mg/ml de métabisulfite de sodium ;
environ 5,0 mg/ml de cétrimide ;
environ 45,0 µg/ml de tartrate d'adrénaline ;
environ 5,0 mg/ml d'hydroxycellulose ;
à 1 ml d'eau purifiée ; et éventuellement un colorant (quantité à adapter) ;
(f) Composition 6 comprenant : lignocaïne, bupivacaïne, adrénaline, cétrimide, 2-hydroxyéthylcellulose, métabisulfite de sodium, sorbitol liquide (70 %), tampon et, éventuellement, colorant ;
(g) Composition 7 dans laquelle la lignocaïne de la Composition 6 est remplacée par de la tétracaïne à raison d'environ 10 mg/ml - 100 mg/ml ;
(h) Composition 8 comprenant : améthocaïne / tétracaïne, adrénaline, cétrimide, 2-hydroxyéthylcellulose, métabisulfite de sodium, sorbitol liquide (70 %), tampon et, éventuellement, colorant ;
(i) Composition 9 comprenant une matrice de gel liquide contenant les éléments suivants :
lidocaïne ; adrénaline ; et
cétrimide,
et la composition est éventuellement colorée, dans laquelle ladite composition a un pH inférieur à environ 4,0;
(j) Composition 10 comprenant une matrice de gel liquide qui contient les éléments suivants : tétracaïne, adrénaline et cétrimide, et la composition est éventuellement colorée, dans laquelle ladite composition a un pH inférieur à environ 4,0; ou
(k) Composition 11 comprenant une matrice de gel liquide qui contient les éléments suivants : lidocaïne, bupivacaïne, adrénaline et cétrimide, et la composition est éventuellement colorée, dans laquelle ladite composition a un pH inférieur à environ 4,0.

3. Composition topique destinée à être utilisée selon la revendication 1 ou la revendication 2, dans laquelle la maladie pathogène est l'ecthyma contagieux (Orf).

4. Composition topique destinée à être utilisée selon la revendication 1 ou la revendication 2, dans laquelle la maladie pathogène est la fièvre aphteuse (FMD).
